# EUROPEAN PATENT APPLICATION

(11) **EP 4 108 673 A1**
(43) Date of publication of application: **28.12.2022**
(21) Application number: 21756343.6
(22) Date of filing: 05.02.2021
(51) Int. Cl.: C07K 1/18, B01D 15/38

(54) **NON-PROTEIN A PURIFICATION METHOD FOR ADALIMUMAB**

(30) Priority: 21.02.2020 KR 20200021721
(71) Applicant: PRESTIGE BIOPHARMA PTE. LTD., Singapore 118222 (SG)
(72) Inventor: YANG, Jae Young, Cheongju-si Chungcheongbuk-do 28160 (KR); PARK, Ji Sung, Cheongju-si Chungcheongbuk-do 28222 (KR); SHIM, Jae Ho, Cheongju-si Chungcheongbuk-do 28160 (KR); UM, Yoon Joeng, Cheongju-si Chungcheongbuk-do 28160 (KR); LEE, Moon Jae, Cheonan-si Chungcheongnam-do 31099 (KR)
(74) Representative: Frenkel, Matthias Alexander
(86) International application number: PCT/KR2021/001519
(87) International publication number: WO 2021/167275

(57) **Abstract**

The present invention relates to a method of preparing a population of antibodies, whereby a desired high-purity and high-quality population of antibodies can be prepared by removing impurities without using an expensive protein A column, and in particular, production costs can be significantly reduced while achieving process automation; and a population of antibodies prepared thereby.

## Description

### TECHNICAL FIELD

The present invention relates to a method of preparing a high-purity and high-yield population of antibodies at low cost without using a protein A column, which is affinity chromatography.

### BACKGROUND ART

The latest trend in biopharmaceutical research and development is more focused on the development of antibody fragments. Although a large number of biosimilar therapeutic proteins have been developed than ever before, significant difficulties in developing biosimilar products are the high costs of downstream processing, nonselective elimination of process and product-related impurities, proteolytic degradation of a product, and the like. Antibody fragments offer certain advantages over full size monoclonal antibody (mAb) therapeutics, such advantages include, for example, enhanced deep penetration into tumors, binding to specific epitopes which are not accessible to full size mAbs, and the like. Advances in upstream processes such as high titer clones and continuous bio-manufacturing have shifted the focus of the biopharmaceutical industry towards improving overall downstream processing economics. The downstream processing constitutes approximately 60 to 70% of the overall manufacturing cost for monoclonal antibody therapeutics. The capture, intermediate and polishing steps of downstream processing involves the use of various chromatographic operations.

Accordingly, a lot of research and development on the purification process has been conducted in relation to antibody drugs.

Specifically, as a purification step for producing an antibody drug, a purification step using a protein A column is mainly performed. However, the purification method using a protein A column has an advantage in that the antibody drug can be produced with high purity at the initial stage, but has a disadvantage in that the production unit price is high because the price is 30-fold higher than that of a general ion exchange resin.

It has already been reported that protein A resin accounts for a high proportion of about 35% of the unit price of antibody drug production raw materials, and a trace amount of protein A which is eluted from the column may cause an immune or physiological reaction inside the human body. Therefore, in the case of a purification process using a protein A column, there are difficulties that the remaining amount of protein A should be monitored and removed for each process. Further, since protein A, which is a biocompatible group, has a disadvantage of being chemically weakly stable, and thus needs to be regenerated while maintaining the activity of protein A at the column regeneration stage, 1 M NaOH, which is essentially used in the cleaning process, cannot be used, so that impurities attached to the column cannot be completely removed, and accordingly, there is a limit that the number of times the column is regenerated and used is significantly reduced compared to other general chemical resins.

In order to solve such problems, many efforts have been made to remove impurities and develop a high-purity antibody using a cation exchange column, a hydrophobic column, an anion exchange column, and the like.

However, in the preparation of an antibody using columns except for the use of protein A as described above, many problems have been discovered up to the preparation of the antibody with high yield and high purity.

For example, when a cation exchange column is generally used, the use of a plurality of buffers is mixed in the purification process. Accordingly, it is difficult to collect proteins, which are eluted, due to a complicated process of using a plurality of buffers. In particular, since a washing process needs to use a buffer composition having a large amount of different components in the use of a cation exchange column, there is a problem in that it is very difficult to achieve automation and process simplification.

Due to the aforementioned problems, antibody production cost and production time have increased significantly, sufficient effects have not been achieved in terms of quality assurance, and process automation has also not been easily achieved.

Against this background, it is very important to provide convenient downstream processing for obtaining biosimilar products, particularly purified antibody fragments. In view of the problems posed by the separation and purification procedures of the related art, the present inventors sought to provide a method of purifying a population of antibodies. A purified population of antibodies obtained by the present invention satisfies and meets excellent purity and activity criteria.

### DISCLOSURE

### TECHNICAL PROBLEM

The present inventors confirmed that after a pre-treatment step of removing a precipitate by reducing the pH of a culture supernatant from which cells had been removed without using an expensive protein A column generally used for antibody purification, a high-quality population of antibodies can be prepared by changing the method as sequentially using a cation exchange column, a hydrophobic interaction column, and an anion exchange column and performing virus filtering and filtration processes at an appropriate time point during this procedure. Accordingly, the present invention provides the invention of the following objects.

One object of the present invention is to provide a method of preparing a population of antibodies, the method including: (a) a step of removing a host cell protein (HCP) and an isomeric antibody from a sample including a mixed solution of antibodies, including loading the sample including the mixed solution of antibodies into an equilibrated cation exchange column, washing the cation exchange column, and then eluting antibodies bound to the column with an elution buffer;
(b) a step of removing the host cell protein (HCP) and the residual DNA from an antibody eluate, including loading a sample obtained by mixing a salt with an antibody eluate eluted in Step (a) into a hydrophobic interaction column and eluting antibodies bound to the column with an elution buffer;
(c) a step of removing the host cell protein (HCP) and the residual DNA, including collecting a flow-through by allowing an antibody eluate from which the host cell protein (HCP) and the residual DNA in Step (b) have been removed to pass through an anion exchange column;
(d) a step of removing viruses by allowing the flow-through in Step (c) to pass through a virus filter; and
(e) a step of concentrating the antibody eluate eluted in Step (d), performing buffer exchange and preparing a population of antibodies containing the residual DNA and the host cell protein at a concentration of 10 ppb and 10 ppm or less, respectively.

Another object of the present invention is to provide a population of antibodies including 60% or more of a main active antibody prepared by the method.

### TECHNICAL SOLUTION

As one aspect to achieve the aforementioned objects, the present invention provides a method of preparing a population of antibodies, the method including: (a) a step of removing a host cell protein (HCP) and an isomeric antibody from a sample including a mixed solution of antibodies, including loading the sample including the mixed solution of antibodies into an equilibrated cation exchange column, washing the cation exchange column, and then eluting antibodies bound to the column with an elution buffer;
(b) a step of removing the host cell protein (HCP) and the residual DNA from an antibody eluate, including loading a sample obtained by mixing a salt with an antibody eluate eluted in Step (a) into a hydrophobic interaction column and eluting antibodies bound to the column with an elution buffer;
(c) a step of removing the host cell protein (HCP) and the residual DNA, including collecting a flow-through by allowing an antibody eluate from which the host cell protein (HCP) and the residual DNA in Step (b) have been removed to pass through an anion exchange column;
(d) a step of removing viruses by allowing the flow-through in Step (c) to pass through a virus filter; and
(e) a step of concentrating the antibody eluate eluted in Step (d), performing buffer exchange and preparing a population of antibodies containing the residual DNA and the host cell protein at a concentration of 10 ppb and 10 ppm or less, respectively.

Antibody products prepared through a host cell include various isomeric antibodies, a host cell protein (HCP), DNA derived from the host cell and factors for cell growth in addition to a main active antibody. The isomeric antibody is an antibody in which some amino acids in the antibody are modified by deamination or oxidation, and biological activity differs depending on the isomeric antibody. Since the specificity of such isomeric antibodies is high in antibody products expressed through host cells and it is important to prepare antibodies of similar quality to control drugs, particularly in the case of antibody biosimilars, antibodies from host cells are produced, and then a process of adjusting the content of isomeric antibodies is required. Thus, the present invention provides a method capable of effectively preparing a high-purity and high-quality population of antibodies including an acidic isomeric antibody, a main active antibody and a basic isomeric antibody at a target ratio by removing a host cell protein and DNA derived from host cells to increase purity and increasing the proportion of the main active antibody compared to a host cell culture solution.

In particular, the method according to the present invention provides a method having not only an advantage in that process automation can be achieved, but also an excellent advantage in that the production unit price is significantly reduced, while providing the high-purity and high-quality population of antibodies described above.

As used herein, the term "a population of antibodies" refers to an antibody group including a main active antibody and an isomeric antibody, and the population of antibodies for the purpose of the present invention refers to an antibody group including a main active antibody and an isomeric antibody at a target ratio. The population of antibodies includes only one type of antibody, or includes all antibody groups including a main active antibody and an isomeric antibody. For the purposes of the present invention, the population of antibodies specifically refers to an antibody group in which the proportion of the main active antibody is increased by removing impurities such as host cell proteins and isomeric antibodies from an antibody product prepared through a host cell.

In particular, when the method of the present invention is applied to the preparation of an antibody biosimilar, a population of antibodies including a main active antibody and an isomeric antibody may be prepared with the same or corresponding composition as that of a control drug.

The target population of antibodies may be prepared so as to include a desired range of isomeric antibodies and main active antibodies by a purification step using a cation exchange resin column, and in this case, specifically, the proportion of the main active antibody may be 50% or more, more specifically, 60% or more, and the proportion of the basic isomeric antibody may be 20% or less, and the proportion of the acidic isomeric antibody may be 20% or less. Specifically, the population of antibodies may include 60% or more of the main active antibody (specifically, 65% or more of the main active antibody), 20% or less of the acidic isomeric antibody, and 20% or less of the basic isomeric antibody.

In the exemplary embodiments of the present invention, a population of antibodies including 20% or less of the acidic isomeric antibody content, 60% or more of the main active antibody and 20% or less of the basic isomeric antibody, which is a content similar to that of Humira control drug quality using a Fractogel COO⁻ cation exchange column.

As used herein, the term "antibody" is a substance produced by stimulation of an antigen in the immune system, and refers to a substance that specifically binds to a specific antigen to cause an antigen-antibody reaction while drifting in lymphatic fluid and blood. For the purposes of the present invention, the antibody is one of the proteins for high quality purification and may be efficiently purified by the method according to the present invention.

Since the antibody generally has a higher isoelectric point than other proteins, the antibody may be primarily purified with relatively high purity when a culture supernatant is adsorbed onto a column and then eluted using an initial cation exchange resin. The isoelectric point (pI) is an average effective charge on the surface of a protein molecule, that is, a pH value at which the potential of the electric double layer of the protein molecule becomes zero, and refers to a point in which the group of the protein is dissociated, the numbers of cations and anions become equal, and thus the effective charge becomes zero. The antibody to be purified in the present invention is not limited thereto and may be an antibody having an isoelectric point of specifically 7 to 10, more specifically, 7 to 9. Further, the antibody of the present invention is not limited thereto, and specifically, may include all therapeutic antibodies typically used in the art. More specifically, the antibody of the present invention may be adalimumab which is a TNF-α inhibitor. The adalimumab is also called Humira, and is a TNF-α inhibitor antibody developed by AbbVie Inc. in the United States, which is known as a therapeutic agent for rheumatoid arthritis, psoriatic arthritis, ankylosing spondylitis, Crohn's disease, ulcerative colitis, psoriasis, and the like.

As used herein, the term "main active antibody" is a major component included in the population of antibodies of the present invention, and refers to an antibody in which some amino acids in the antibody are modified by deamination or oxidation, and thus biological activity is not lowered, that is, an antibody which is not an acidic or basic isomeric antibody. The main active antibody is the most important component for adjusting the quality of a target population of antibodies, and is an antibody with the highest biological activity among the components of the antibody.

As used herein, the term "isomeric antibody" refers to an antibody in which some amino acids of the main active antibody are modified by deamination or oxidation, and includes acidic isomeric antibodies and basic isomeric antibodies. Examples thereof include isomeric antibodies in which asparagine, among amino acids, is deaminated to become aspartate, and isomeric antibodies in which methionine, among amino acids, is oxidized to become methionine sulfate. In addition, when glutamate is present at the N-terminus of a heavy chain, the glutamate forms a pentagonal ring structure to include an isomeric antibody modified into pyruglutamate. When isomeric antibodies are produced from host cells such as CHO cells, the isomeric antibodies are included in a host cell culture solution at a high proportion, so the isomeric antibodies are removed by a process such as chromatography, and need to be included in the population of antibodies at a desired proportion.

Therefore, in order to prepare a high-quality population of antibodies in a host cell into which a vector including a polynucleotide encoding an antibody has been introduced, it is necessary to appropriately remove the aforementioned isomeric antibody, so that the main active antibody and the isomeric antibody are included at a desired content. Furthermore, in order to prepare a high-purity population of antibodies, impurities such as a host cell protein (HCP), host cell-derived DNA (HCD) and factors for cell growth need to be removed. Thus, in the present invention, a method of preparing a population of antibodies in which impurities such as a host cell protein are effectively removed, in addition to adjusting the content of the isomeric antibody was developed.

In particular, the method according to the present invention provides a method having an excellent advantage in that not only process automation can be achieved, but also the production unit price is significantly reduced, while providing the high-purity and high-quality population of antibodies described above. Further, in terms of virus inactivation and removal ability, when the purification process according to the present invention is used, even though there is unexpected virus expression or contamination, it can be removed.

The method of preparing a population of antibodies according to the present invention includes: (a) a step of removing a host cell protein (HCP) and an isomeric antibody from a sample including a mixed solution of antibodies, including loading the sample including the mixed solution of antibodies into an equilibrated cation exchange column, washing the cation exchange column, and then eluting antibodies bound to the column with an elution buffer.

The method in Step (a) is a step of collecting an antibody eluate including an antibody in which a host cell protein and an isomeric antibody have been removed from a sample including a mixed solution of antibodies.

As used herein, the term "sample including a mixed solution of antibodies" is a sample partially purified from a culture supernatant of cells producing an antibody or a lysate of the cells, and refers to a partially purified sample including a mixed solution of antibodies including both a main active antibody and an isomeric antibody. The partial purification performed a filtration process, but refers to a state in which other proteins other than the antibody are also present.

The sample including the mixed solution of the antibody is characterized by being prepared by sequentially performing a step of preparing a culture supernatant by culturing host cells to produce a target antibody and removing the host cells; and a step of removing a precipitated precipitate by adjusting the pH of the culture supernatant to a pH lower than an isoelectric point of the target antibody, specifically a pH of 4 to 6. That is, a sample including a mixed solution of antibodies may be prepared by a method including a step of removing a precipitated precipitate by adjusting the pH of the culture supernatant to 4 to 6.

The case where a sample is prepared by reducing the pH and then removing cells has a disadvantage in that the death of the cells is promoted by the decrease in pH to increase the content of a host cell protein. Accordingly, the method of preparing a sample by removing cells and then adjusting the pH of the culture supernatant is more advantageous for lowering the content of the host cell protein. Therefore, in the present invention, specifically, a sample prepared by a method of preparing a sample, in which cells are removed and then the pH of the culture supernatant is adjusted, is used.

As the method for removing cells, a method typically used in the art can be used, and although not being limited thereto, specifically a filter, more specifically, a filtration filter may be used.

The sample including the mixed solution of antibodies may be adjusted so as to have a conductivity of 5 to 7 mS/cm before being injected into a cation exchange column. In exemplary embodiments of the present invention, conductivity was adjusted by adding purified water to a pre-treated supernatant before injection into a column.

According to the present invention, through the process of removing cells from the culture solution through a primary filtration filter, lowering the recovered culture supernatant to a pH of 5 again and re-filtering the culture supernatant, the content of the host cell protein was significantly low and even a turbidity analysis exhibited an excellent effect.

In addition, a precipitate formed by reducing the pH contains a large amount of host cell protein, so when the host cell protein is removed, the content of the host cell protein may be lowered. The removal of the precipitate is a previous step for adsorbing the antibody onto the cation exchange column, and the antibody does not aggregate under acidic conditions with a low pH because the isoelectric point thereof is high, whereas in general, in the case of host cell proteins (HCPs) having a lower isoelectric point than antibodies, the charge is removed under acidic conditions with a lowered pH, so that a large amount of host cell protein may be precipitated because the host cell protein is aggregated by the van der Waals force. Therefore, the reduced pH range of the culture supernatant may include a value which is lower than the isoelectric point of an antibody to be purified in the present invention and simultaneously close to the isoelectric point of the host cell protein in order to increase the precipitation of the host cell protein to be removed. That is, the pH may be a value which is specifically 1 to 6 smaller, more specifically 2 to 5 smaller than the isoelectric point of the antibody to be purified. Therefore, the pH may be finally specifically 3 to 7, more specifically 4 to 6, and even more specifically 4.5 to 5.5. In this case, the precipitate may be removed using a filter typically used in the art such as a sterilization filter. In exemplary embodiments of the present invention, it was confirmed that when the precipitate is removed by reducing the pH to 5, a pre-treatment step in which purity is high could be performed. When the precipitate is removed by the pre-treatment step described above, the population of antibodies may be more efficiently purified in a subsequent purification step using a cation exchange column, a hydrophobic interaction column, and an anion exchange column.

In Step (a), the sample including the mixed solution of antibodies prepared as described above is injected into a cation exchange column, and the cation exchange column is washed to remove an isomeric antibody and a host cell protein, and then antibodies bound to the column are eluted.

As used herein, the term "cation exchange column" refers to a column packed with a cation exchange resin, and in the step, impurities, specifically the host cell protein and the isomeric antibody, may be removed by performing cation exchange chromatography. The cation exchange resin is a synthetic resin that serves to exchange cations in an aqueous solution with its own cations, and since an antibody has a high isoelectric point, a pH buffer solution having an isoelectric point value or less becomes positively charged. Therefore, the quality of the population of antibodies may be improved using a cation exchange resin capable of adsorbing the cation-bearing antibody. As the cation exchange resin, those typically used in the art may be used, and although not limited thereto, specifically, a column having a functional group of COO⁻ or SO₃ may be used, and more specifically, carboxymethyl (CM), Fractogel, sulfoethyl (SE), sulfopropyl (SP), phosphate (P), sulfonate (S) or the like may be used, and even more specifically, Fractogel SO₃⁻ (s), POROS XS, POROS HS, carboxymethyl sepharose (CM sepharose) or Fractogel COO⁻ may be used.

Step (a) is characterized by simultaneously removing a host cell protein and an isomeric antibody of a target antibody.

In this case, the host cell protein may include all impurities except for the antibody to be purified, and may include not only the host cell protein itself but also all of DNA derived from the host cells, factors for cell growth and the like. Therefore, when the host cell protein is removed, only the antibody to be purified may be purified with high purity.

In addition, the step is characterized by being a step of removing the isomeric antibody for quality control. The isomeric antibody may be an acidic isomeric antibody and/or a basic isomeric antibody. Since various isomeric antibodies are present in an antibody product expressed through host cells, it is important to make the quality most similar to that of a control drug in order to prepare an antibody biosimilar. As the isomeric antibodies are variants in which some amino acids of the main active antibody are modified, there is a subtle difference in charge among the main active antibody, the acidic isomeric antibody and the basic isomeric antibody. Therefore, the isomeric antibody may be separated using this charge difference. However, since the charge difference is simply a subtle difference caused by some amino acids, very detailed conditions for separation need to be set. Therefore, in the present invention, a cation exchange column is used for effective removal of the acidic isomeric antibody and the basic isomeric antibody. That is, the method according to the present invention also has an excellent advantage in that a charge variant can be adjusted at a desired ratio using a cation exchange column.

Furthermore, the isomeric antibodies are obtained by modifying some amino acids in the antibody by deamination or oxidation, and since it is known that biological activity differs depending on the isomeric antibody, it is important to keep the content distribution of the isomeric antibodies constant in order to maintain a consistent quality. However, since a host cell culture supernatant, which produces an antibody, generally has a relatively high content of acidic and basic isomeric antibodies compared to the main active antibody, the contents of three types of antibodies need to be adjusted by partially removing antibodies, and in the present invention, a population of antibodies is prepared such that the proportion of the main active antibody in population of antibodies is 50%, specifically, 60% or more. Further, the method of the present invention may prepare the population of antibodies such that the proportion of the main active antibody, the proportion of the basic isomeric antibody, and the proportion of the acidic isomeric antibody are 60% or more, 20% or less, and 20% or less, respectively.

In Step (a), in the loading of the sample including the mixed solution of antibodies into the equilibrated cation exchange column, an equilibration buffer having a pH of 4.5 to 5.5 and including 15 mM to 30 mM acetate and 35 mM to 45 mM sodium chloride may be used.

Specifically, the loading of the sample including the mixed solution of antibodies into the equilibrated cation exchange column may be a step of loading a mixed solution of antibodies into a Fractogel COO⁻ cation exchange column equilibrated with an equilibration buffer having a pH of 4.5 to 5.5 and including 15 mM to 30 mM acetate and 35 mM to 45 mM sodium chloride. The equilibration buffer may have a conductivity of 5 to 7 mS/cm, specifically 5.5 to 6.5 mS/cm, and more specifically about 6.0 mS/cm. In the case of such equilibration, it is desirable to use the buffer in an amount of about 11 column volumes or more, specifically 14 column volumes.

According to exemplary embodiments of the present invention, the purification process was initiated with a cation exchange column by loading a sample including a mixed solution of antibodies into a Fractogel COO⁻ cation exchange column equilibrated with an equilibration buffer having a conductivity of 6.0 mS/cm and a pH of 5.5 and including 20 mM acetate and 40 mM sodium chloride.

In Step (a), the washing step may include two steps of, specifically, a first washing step of attaching an unattached antibody to a column; and a second washing step of removing an acidic isomeric antibody.

According to the existing method of using a cation exchange column, the composition, pH and conductivity of the buffer used in each step were significantly different while including multiple washing steps.

In contrast, in the present invention, the unit price may be reduced and the manufacturing process may be automated in terms of the manufacturing process by using only two washing steps and a buffer having a simple composition.

Specifically, the washing step according to the present invention may consist of: a step of performing a first washing with a buffer having a pH of 4.5 to 5.5 and including 15 mM to 30 mM acetate and 35 mM to 45 mM sodium chloride; and 2) a step of performing a second washing with a buffer having a pH of 5.5 to 6.5 and including 25 mM to 35 mM acetate and 55 to 59 mM, specifically 57 mM sodium chloride.

The first washing process in the present invention may perform a first washing with a buffer having a pH of 4.5 to 5.5 and including 15 mM to 30 mM acetate and 35 mM to 45 mM sodium chloride, which includes the same components as the buffer used in the loading step. Accordingly, the first washing step has an advantage in that an antibody that is not attached to the column can be additionally attached through a continuous process without the need for a separate buffer preparation process. In the case of such a first washing, it is desirable to use the buffer in an amount of about 3 to 7 column volumes, specifically about 5 column volumes.

The second washing process in the present invention may perform a second washing with a buffer having a pH of 5.5 to 6.5 and including 25 mM to 35 mM acetate and 55 to 59 mM, specifically, 57 mM sodium chloride.

In the second washing process and the subsequent elution (desorption) process according to the present invention, purification is performed in a cation exchange column while varying the treatment ratio of the two buffers. Unlike repeating the washing and equilibration processes or performing four or more consecutive washing processes in buffers with different compositions in the existing related art, in the present invention, the purification process in Step (a) may be conveniently performed by adjusting only the ratio of the two buffers. Accordingly, the second washing process and the subsequent elution (desorption) process have an advantage of being able to achieve unit price reduction and simplification of the manufacturing process in terms of the manufacturing process.

That is, in the present invention, the washing and elution process in Step (a) may be performed while constantly including a 25 mM to 35 mM acetate buffer having a pH of 5.5 to 6.5 and increasing the molar concentration of sodium chloride. That is, the washing and desorption process is performed by increasing the molar concentration of sodium chloride while changing (increasing) the mixture ratio of a buffer having a pH of 5.5 to 6.5 and including 25 mM to 35 mM acetate and a buffer having a pH of 5.5 to 6.5 and including 25 mM to 35 mM acetate and sodium chloride having a predetermined molar concentration.

For example, according to exemplary embodiments of the present invention, in the second washing process, it is possible to use a mixture of a first buffer having a pH of 5.5 to 6.5 and including 25 mM to 35 mM acetate; and a second buffer having a pH of 5.5 to 6.5 and including 25 mM to 35 mM acetate and 100 mM sodium chloride. Specifically, a buffer composition required for the second washing process may be made by mixing 41 to 45 wt% of a first buffer having a pH of 5.5 to 6.5 and including 25 mM to 35 mM acetate; and 55 to 59 wt% of a second buffer having a pH of 5.5 to 6.5 and including 25 mM to 35 mM acetate and 100 mM sodium chloride. Such mixing may be performed by providing an appropriate volume of each buffer from each of the two buffer storage spaces according to the mixture ratio of each step set in the device.

In the second washing step according to a specific embodiment of the present invention, the second washing process was performed using a buffer having a pH of 5.5 to 6.5 and including 30 mM acetate and 57 mM sodium chloride by treatment with 43 wt% of the first buffer and 57 wt% of the second buffer. In the case of such a second washing, it is desirable to use the buffer in an amount of about 3 to 17 column volumes, specifically about 15 column volumes or before an AU of 0.1 is exhibited.

In Step (a), the elution (desorption) step may include three steps of, specifically, a first elution step; a second washing step; and a third elution step.

Specifically, the elution (desorption) step may consist of 1) a first elution step of eluting an antibody with a buffer including having a pH of 5.5 to 6.5 and including 25 mM to 35 mM acetate and 57 to 63 mM, specifically, 60 mM sodium chloride; 2) a second elution step of eluting the antibody with a buffer having a pH of 5.5 to 6.5 and including 25 mM to 35 mM acetate and 67 to 73 mM, specifically 70 mM sodium chloride; and 3) a third elution step of eluting the antibody with a buffer having a pH of 5.5 to 6.5 and including 25 mM to 35 mM acetate and 77 to 83 mM, specifically 80 mM sodium chloride.

In the present invention, as previously confirmed in the second washing process, the elution (desorption) step may be conveniently performed by adjusting only the ratio of the two buffers for treating the elution process. Accordingly, unit price reduction and automation of the manufacturing process have been achieved in terms of the manufacturing process.

The elution process in Step (a) may be performed while constantly including a 25 mM to 35 mM acetate buffer having a pH of 5.5 to 6.5 and increasing the molar concentration of sodium chloride. That is, the desorption process is performed by increasing the molar concentration of sodium chloride while changing (increasing) the mixture ratio of a buffer having a pH of 5.5 to 6.5 and including 25 mM to 35 mM acetate and a buffer having a pH of 5.5 to 6.5 and including 25 mM to 35 mM acetate and sodium chloride having a predetermined molar concentration.

For example, according to exemplary embodiments of the present invention, the buffer used in the elution process may be a mixture of a first buffer having a pH of 5.5 to 6.5 and including 25 mM to 35 mM acetate; and a second buffer having a pH of 5.5 to 6.5 and including 25 mM to 35 mM acetate and 100 mM sodium chloride.

Specifically, in the first elution process, the buffer may include 37 to 43 wt% of a first buffer having a pH of 5.5 to 6.5 and including 25 mM to 35 mM acetate; and 57 to 63 wt% of a second buffer having a pH of 5.5 to 6.5 and including 25 mM to 35 mM acetate and 100 mM sodium chloride.

Specifically, in the second elution process, the buffer may include 27 to 33 wt% of a first buffer having a pH of 5.5 to 6.5 and including 25 mM to 35 mM acetate; and 67 to 73 wt% of a second buffer having a pH of 5.5 to 6.5 and including 25 mM to 35 mM acetate and 100 mM sodium chloride.

Specifically, in the third elution process, the buffer may include 17 to 23 wt% of a first buffer having a pH of 5.5 to 6.5 and including 25 mM to 35 mM acetate; and 77 to 83 wt% of a second buffer having a pH of 5.5 to 6.5 and including 25 mM to 35 mM acetate and 100 mM sodium chloride.

In the first elution step according to a specific embodiment of the present invention, the first elution process was performed using a buffer having a pH of 5.5 to 6.5 and including 30 mM acetate and 60 mM sodium chloride by treatment with 40 wt% of the first buffer and 60 wt% of the second buffer. In the case of such a first elution, it is desirable to use the buffer in an amount of about 5 to 20 column volumes, specifically about 7 to 17 column volumes, and more specifically about 8 to 15 column volumes.

In the second elution step according to a specific embodiment of the present invention, the second elution process was performed using a buffer having a pH of 5.5 to 6.5 and including 30 mM acetate and 70 mM sodium chloride by treatment with 30 wt% of the first buffer and 70 wt% of the second buffer. In the case of such a second elution, it is desirable to use the buffer in an amount of about 7 to 20 column volumes, specifically about 8 to 17 column volumes, and more specifically 9 to 15 column volumes.

In the third elution step according to a specific embodiment of the present invention, the third elution process was performed using a buffer having a pH of 5.5 to 6.5 and including 30 mM acetate and 80 mM sodium chloride by treatment with 20 wt% of the first buffer and 80 wt% of the second buffer. In the case of such a third elution, it is desirable to use the buffer in an amount of about 6 to 18 column volumes, specifically about 8 to 16 column volumes, and more specifically 9 to 14 column volumes or up to an AU of 0.1.

That is, (a) a step of removing a host cell protein (HCP) and an isomeric antibody from a sample including a mixed solution of antibodies, including loading the sample including the mixed solution of antibodies into an equilibrated cation exchange column, washing the cation exchange column, and then eluting antibodies bound to the column with an elution buffer, may specifically include the following in the loading, washing and elution step:
1) a step of loading a mixed solution of antibodies into a Fractogel COO⁻ cation exchange column equilibrated with an equilibration buffer having a pH of 4.5 to 5.5 and including 15 mM to 30 mM acetate and 35 mM to 45 mM sodium chloride;
2) a first washing step of washing the column with a buffer having a pH of 4.5 to 5.5 and including 15 mM to 30 mM acetate and 35 mM to 45 mM sodium chloride;
3) a second washing step of washing the column with a buffer having a pH of 5.5 to 6.5 and including 25 mM to 35 mM acetate and 55 to 59 mM, specifically 57 mM sodium chloride;
4) a first elution step of eluting an antibody with a buffer having a pH of 5.5 to 6.5 and including 25 mM to 35 mM acetate and 57 to 63 mM, specifically 60 mM sodium chloride;
5) a second elution step of eluting the antibody with a buffer having a pH of 5.5 to 6.5 and including 25 mM to 35 mM acetate and 67 to 73 mM, specifically 70 mM sodium chloride; and
6) a third elution step of eluting the antibody with a buffer having a pH of 5.5 to 6.5 and including 25 mM to 35 mM acetate and 77 to 83 mM, specifically 80 mM sodium chloride.

In the exemplary embodiments of the present invention, antibodies having a high content of the main active antibody were purified while significantly reducing host-derived protein and DNA by purifying the antibody through the steps of equilibration and loading; a first washing; a second washing; a first elution; a second elution; and a third elution including the above steps.

In the above method, in the method of removing an isomeric antibody, specifically, Fractogel COO⁻ may be used in order to simultaneously remove an acidic isomeric antibody and a basic isomeric antibody.

When a large amount of basic isomeric antibody is mixed with the acidic isomeric antibody, a cation exchange column having a separation ability is also required to remove the basic isomeric antibody, and in this case, it is desirable to simultaneously remove the acidic isomeric antibody and the basic isomeric antibody using Fractogel COO⁻ consisting of a methacrylate polymer resin which is a synthetic polymer as a support.

The cation exchange chromatography according to the present exemplary embodiment has an advantage in that, as the flow rate, it is also possible to use a flow rate which is 1.5-fold or faster (for example, a flow rate which is 1.55-fold to 2.24-fold faster) than the 180 cm/hr of existing methods. In the case of such a high flow rate, it is possible to have a higher production rate over time than that of the existing method.

In the present invention, after Step (a), it is possible to include a step of inactivating viruses before performing Step (b).

Specifically, the virus inactivation includes rendering viruses contained in the eluate non-functional or removing viruses from the eluate. A method of rendering viruses non-functional or removing viruses includes heat inactivation, pH inactivation, chemical inactivation, or the like, and specifically, a pH inactivation method may be used, but the method is not limited thereto. The pH inactivation method is a method of treating viruses with a pH at which the viruses become sufficiently non-functional, and such a pH inactivation method includes a low-pH virus inactivation method, and the method may be performed by titrating the antibody eluate eluted in Step (a) in a pH range of 3.0 to 4.0, specifically, at a pH of 3.8, but is not limited thereto.

Specifically, the antibody eluate eluted in Step (a) may include 60% or more of the main active antibody, 20% or less of the acidic isomeric antibody, and 20% or less of the basic isomeric antibody.

The method of preparing a population of antibodies according to the present invention includes: (b) a step of removing the host cell protein (HCP) and the residual DNA from an antibody eluate, including loading a sample obtained by mixing a salt with an antibody eluate eluted in Step (a) into a hydrophobic interaction column and eluting antibodies bound to the column with an elution buffer.

In the method of the present invention, the method of Step (b) is a step of collecting the antibody eluate from which the host cell protein and the residual DNA have been further removed from the antibody eluate collected in Step (a).

The antibody eluate collected in Step (a) may be the collected eluate itself or in the form of being additionally diluted with another buffer. Further, as mentioned above, the eluate may also be an eluate sample that has undergone the step of inactivating viruses. In the present invention, the sample loaded into the hydrophobic interaction column may be prepared by adding a salt to the antibody eluate eluted in Step (a). Although the type of salt included in the sample loaded into the hydrophobic interaction column is not particularly limited, citrate was used in exemplary embodiments of the present invention. In addition, the sample may be a sample adjusted to have a salt concentration of 0.8-fold to 1.2-fold the salt concentration of the equilibration buffer of the hydrophobic interaction column in Step (b), specifically, may be a sample adjusted to have the salt concentration of the antibody eluate in Step (a), which is the same as the salt concentration of the equilibration buffer, but is not limited thereto.

Furthermore, the elution step of Step (b) includes eluting the antibody attached to the column by a concentration gradient method. In exemplary embodiments of the present invention, it was confirmed that the concentration gradient method is more advantageous in terms of yield and elution volume than the step method.

Step (b) has a purpose of further increasing purity by removing impurities such as a host cell protein and the residual DNA which could not be removed in Step (a), and provides a purification step using a hydrophobic interaction column capable of removing the host cell protein, which has a separation mechanism different from that of the cation exchange column of (a) purification step.

As used herein, the term "hydrophobic interaction column" refers to a column packed with a hydrophobic interaction resin, and refers to a column capable of removing impurities, specifically the host cell protein, by performing hydrophobic interaction chromatography in the above step. Although proteins are generally hydrophilic, there are regions that are hydrophobic as well as hydrophilic, and the hydrophobic properties of these regions are not expressed under conditions of strong electrostatic interaction, but are characterized by being relatively strongly expressed when the electrostatic interaction is weakened by increasing the ionic strength or dielectric constant of the solvent. Here, when a hydrophobic ligand (long hydrocarbon chain or aromatic ring) is introduced into a substrate for hydrophilic chromatography (agarose gel fizz, organic polymer support, and the like) and equilibrated to a high salt concentration, various proteins may be adsorbed, and thereafter, when the salt concentration is lowered, proteins may be separated because the proteins are eluted depending on the properties of the proteins. That is, when a hydrophobic environment is provided using a salt, a difference in the hydrophobicity of each protein causes a difference in the strength of being adsorbed onto a specific column, and Step (b) of removing the host cell protein and the residual DNA using a hydrophobic interaction column may be performed using such a principle.

As the hydrophobic interaction resin, those typically used in the art may be used, but the hydrophobic interaction resin is not limited thereto, and specifically, a phenyl column, a butyl column, a phenyl sepharose or a Fractogel EMA phenyl column, and the like may be used, and more specifically, phenyl sepharose may be used.

Further, Step (b) may include a step of eluting an antibody by loading a sample in which the antibody eluate eluted in Step (a) is adjusted to a citrate concentration that is the same as that of an equilibration buffer into a hydrophobic interaction column equilibrated with an equilibration buffer including 25 mM to 35 mM acetate (pH of 5.5 to 6.5) and 0.3 M to 1.0 M citrate and applying an elution buffer including 25 mM to 35 mM acetate (pH of 5.5 to 6.5) in a concentration gradient manner.

In the present invention, it was confirmed that when an acetate buffer under a condition of pH 6.0 was used as the buffer, the yield was high, and even in the elution method, by confirming that a method of using the concentration gradient of the buffer has an excellent yield, it was confirmed that the above step of the present preparation method using the concentration gradient method was useful for the preparation of a high-purity population of antibodies.

The method of preparing a population of antibodies according to the present invention may include filtering the antibody eluate in Step (b) using a filter after Step (b) and before Step (c).

In the present invention, the filtration may be, for example, ultrafiltration and/or diafiltration.

Specifically, ultrafiltration may be performed. As used herein, the term "ultrafiltration" or "UF" refers to any technique for treating a solution or suspension with a semi-permeable membrane that retains macromolecules while allowing a solvent or small solute molecules to pass through. Ultrafiltration may be used to increase the concentration of macromolecules in a solution or suspension.

In addition, specifically, diafiltration may be performed. As used herein, the term "diafiltration" or "DF" is used to mean a specialized filtration category that dilutes a retentate with a solvent and refilters the retentate in order to reduce soluble permeate components. Diafiltration may induce or not induce an increase in the concentration of, for example, retained components including proteins. For example, in continuous diafiltration, the solvent is continuously added to the retentate at the same rate as the production rate of the filtrate. In this case, the volume of the retentate and the concentration of the retained components do not change during the process. Meanwhile, in discontinuous or sequentially diluted diafiltration, the ultrafiltration step involves the addition of a solvent to the retentate side; when the volume of solvent added to the retentate is equal to or greater than the volume of the resulting filtrate, the retained components will have a high concentration. Diafiltration may be used to modify pH, ionic strength, salt composition, buffer composition, or other characteristics of a solution or suspension of macromolecules.

Through such a first filtration process, the content of host cell protein may be further reduced.

The method of preparing a population of antibodies according to the present invention includes (c) a step of removing the host cell protein (HCP) and the residual DNA, including collecting a flow-through by allowing an antibody eluate from which the host cell protein (HCP) and the residual DNA in Step (b) have been removed to pass through an anion exchange column.

In the method of the present invention, the method in Step (c) is a step of collecting a desired population of antibodies in which impurities have been removed from an antibody eluate collected in Step (b), and specifically, Step (c) is a step of collecting a flow-through by allowing the host cell protein (HCP) and the residual DNA to pass through an anion exchange column. In addition, the antibody eluate collected in Step (b) may be the collected eluate itself, in the form of being additionally diluted with another buffer, in the form in which a filtration process or the like is additionally performed, or the like, but is not limited thereto.

As used herein, the term "anion exchange column" refer to a column packed with an anion exchange resin, and refers to a column capable of removing impurities, specifically the host cell protein, by performing anion exchange chromatography in the above step, but is not limited thereto. The anion exchange resin is a synthetic resin that serves to exchange a specific anion in an aqueous solution with its own anion, and the cation exchange column may adsorb an anion-bearing protein at an isoelectric point or higher. Since an antibody has a high isoelectric point, the antibody escapes without being attached to the anion exchange resin when a neutral pH buffer is used, but impurities including a host cell protein have a low isoelectric point, and thus may be removed while being adsorbed onto the anion exchange resin, so the anion exchange resin may be used for preparing a high-purity population of antibodies using the above principle.

As the anion exchange resin, those typically used in the art may be used, but the anion exchange resin is not limited thereto, and specifically, Q sepharose, quaternary aminoethyl, quaternary amine (Q), or the like may be used, and more specifically, Q Fast Flow may be used.

Furthermore, the method may use an equilibration buffer having a pH lower than the pI of a target antibody, specifically an equilibration buffer having a pH of 7.0 to 8.0, and more specifically, an equilibration buffer including Tris-hydrogen chloride (pH of 7.0 to 8.0).

The host cell protein removed in the above step is a concept that includes all impurities except for the antibody to be purified as mentioned above, and may include not only the host cell protein itself but also all of DNA derived from the host cells, factors for cell growth and the like. Therefore, when the host cell protein is removed in the present step, only the antibody to be purified may be purified with high purity. Further, since the anion exchange column is an efficient column for removing not only the host cell protein but also endotoxins, a target population of antibodies having high purity may be purified by removing an endotoxin together with the host cell protein in the final purification step.

The method of preparing a population of antibodies according to the present invention includes (d) a step of removing viruses by allowing the flow-through in Step (c) to pass through a virus filter.

In the method of preparing a population of antibodies according to the present invention, virus filtering is performed before performing the filtration using ultrafiltration, diafiltration and/or a multi-layer filtration filter or the like unlike general methods in the existing related art. In the existing general methods, it is difficult to prepare a high-concentration drug because viruses are filtered after filtration is performed using ultrafiltration, diafiltration, and a multi-layer filtration filter. In contrast, the method according to the present invention has an advantage in that by first performing virus filtering to reduce the loading amount in the ultrafiltration, diafiltration and/or multi-layer filtration filtering step, protein quality is secured and simultaneously the yield loss is lowered, and a high-concentration drug is easily prepared.

Specifically, in the present invention, Modus 1.3 (product from Merck) may be used as a virus filter. Specifically, it is desirable to use a PES material as the corresponding material.

The method of preparing a population of antibodies according to the present invention includes (e) a step of concentrating the antibody eluate eluted in Step (d), performing buffer exchange and preparing a population of antibodies containing the residual DNA and the host cell protein at a concentration of 10 ppb and 10 ppm or less, respectively.

In the present invention the concentrating of the prepared antibody eluate and the performing of buffer exchange may mean a typical concentration and buffer exchange process for storage of antibodies.

The solution (eluted antibody eluate) including the filtered antibody subjected to the above virus filtering may be subsequently subjected to ultrafiltration, diafiltration and the like. Ultrafiltration and diafiltration are the same as described above.

Through the virus removal and filtration step as described above, virus impurities may be removed and the HCP and the residual DNA may be further removed. In addition, such subsequent ultrafiltration and diafiltration may be used for concentration and/or buffer exchange and the like.

A buffer may store a typical antibody or include all the components having the buffer requirements required for preparing a formulation. For example, the buffer is an example of a pharmaceutically acceptable excipient, and may include a general buffer component for preparing a formulation having an excipient(s) having a known concentration by including any non-ionic excipient(s) or ionic excipient(s) shown in a document [Reference: Remington's Pharmaceutical Sciences 16th edition, Osol, A. Ed. (1980)] cited as a reference in the present invention as an example of a pharmaceutically acceptable excipient. The buffer may be exchanged with a buffer including a non-ionic excipient, for example, a sugar such as polysorbate and a poloxamer or a non-ionic surfactant without changing the antibody concentration or the like.

The population of antibodies according to the present invention prepared according to the present invention and subjected to the concentration and buffer exchange may have a host cell protein content of 10 ppm or less, for example, 0.0001 to 10 ppm, and more particularly 0.001 to 5 ppm. Furthermore, the content of the residual DNA may be 10 ppb or less, for example 0.0001 to 10 ppb, more specifically 0.001 to 1 ppb.

In exemplary embodiments according to the present invention, it was confirmed that an antibody could be purified with a high purity of 99.9% by the antibody purification method of the present invention. In particular, the prepared population of antibodies included a population of antibodies containing the residual DNA and the host cell protein at a concentration of 0.1 ppb and 5 ppm or less, respectively.

As another aspect, the present invention provides a population of antibodies including 60% or more of a main active antibody prepared by the method.

The above method, the population of antibodies, and the population of antibodies containing 60% or more of the main active antibody are as described above.

### ADVANTAGEOUS EFFECTS

When the method of preparing a population of antibodies according to the present invention is used, it is possible to prepare a target population of antibodies with high purity and high quality by removing impurities without using an expensive protein A column. Furthermore, the method according to the present invention provides a method having an excellent advantage in that process automation is achieved and the production unit price is significantly reduced.

### DESCRIPTION OF DRAWINGS

FIG. 1 is a flow chart illustrating a process of preparing an adalimumab antibody according to the present invention.
FIG. 2 illustrates the results of cation exchange chromatography performed by a method similar to the method described in Korean Patent No. 10-1498771.
FIG. 3 illustrates the results of performing cation exchange chromatography according to Example 2-1 of the present invention.
FIG. 4 illustrates the results of performing cation exchange chromatography according to Example 2-2 of the present invention.
FIG. 5 illustrates the results of performing hydrophobic interaction chromatography according to the present invention.
FIG. 6 illustrates the results of performing anion exchange resin chromatography according to the present invention.

### MODES OF THE INVENTION

Hereinafter, the present invention will be described in detail through the Examples. However, the following Examples are only for exemplifying the present invention, and the present invention is not limited by the following Examples.

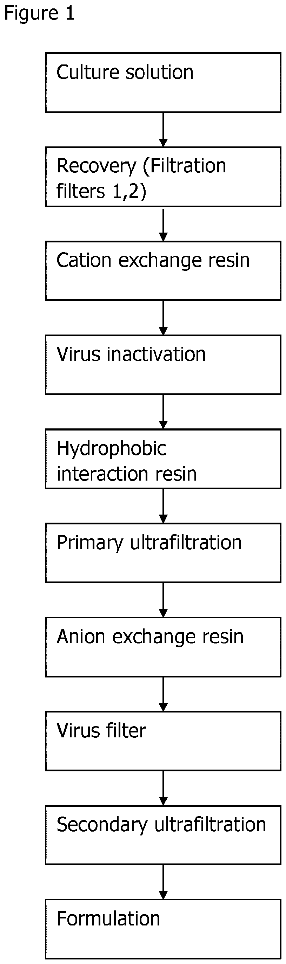

Hereinafter, the flow chart of the antibody preparation process according to exemplary embodiments is specifically shown in FIG. 1. Specifically, a culture solution sample including the antibody according to the present invention is recovered, filtered, and then allowed to pass through a cation exchange resin. Then, the sample is subjected to virus inactivation and allowed to pass through a hydrophobic interaction resin. Next, the sample is subjected to primary ultrafiltration, and then allowed to pass through an anion exchange resin. Finally, the sample is allowed to pass through a virus filter and subjected to secondary ultrafiltration, and then formulation is performed. The specific processes briefly described above will be specifically described in the following examples.

### Example 1: Pre-treatment method of culture solution for antibody purification

After an adalimumab antibody was expressed by culturing recombinant CHO cells expressing the adalimumab antibody, pH was lowered to 6 or less in order to adsorb the antibody onto a cation exchange column.

In the present example, the degree of removal of impurities was confirmed by the pre-treatment method of a culture solution.

In the present example, a method of preparing a sample for injecting into a cation exchange column through a filtration filter after lowering the pH to 5 in a state of a culture solution including cells was used, and the specific conditions thereof are shown in Table 1.

**[Table 1]**

| Proce dure | Method |
|---|---|
| 1 | Culture solution |
| 2 | Supernatant is recovered by removing cells with a first filtration filter (Depth Filter) and a second filtration filter (sterilization filter) |
| 3 | Adjust conductivity |
| 4 | pH is lowered to 5 by adding 10% acetic acid to supernatant |
| 5 | Stir at low speed at room temperature (about 25°C) for 1 hour |
| 6 | Precipitate is removed and bacteria are sterilized with filtration and sterilization filters |

The culture solution is pre-treated by the above method, and turbidities of the culture solution of Procedure (1), the supernatant of Procedure (2), the culture solution after acid treatment of Procedure (4), and the pre-treatment culture solution after filtration and sterilization filters of Procedure (6) were analyzed. As a result, in the case of the culture solution of (1), the turbidity was confirmed to be 6810 NTU, and in the case of the supernatant of (2), the turbidity was confirmed to be 2.98 NTU. Further, in the case of the culture solution after acid treatment of (4), the turbidity was confirmed to be 5475 NTU, and in the case of the pre-treated culture solution after filtration and sterilization filters of final (6), the turbidity was confirmed to be 5.13 NTU.

In addition, it could be confirmed that the average flux showed a large filtration capacity around 150 LMH in the filtration step, and thus loss was small in the pre-treatment stage of the culture solution.

It was confirmed that a pre-treated culture solution suitable for use in the subsequent steps was prepared by showing a turbidity removal rate of about 99% or more after the filtration treatment at each step.

The results show that a method of performing a process after primary removal of cells with an initial filtration filter as the pre-treatment method of the present invention is suitable. In addition, the results showed that when the precipitate was removed by lowering the pH to 6 or less (preferably pH 5), a culture supernatant having a much higher purity than the initial culture solution could be obtained.

### Example 2: Cation exchange chromatography

Among the cation exchange columns capable of replacing a column process using Protein A, Fractogel COO⁻ was selected as a column having a functional group advantageous in terms of purity and yield.

An experiment was performed to adjust an isomeric antibody using the cation exchange column set above, and the process thereof is as follows.

For equilibration, the column was equilibrated (6.0 mS/cm) by flowing an amount of 14 column volumes of a buffer having a pH of 5.0 and including 20 mM and 40 mM sodium chloride, and then the pre-treated supernatant was loaded below the adsorption capacity of CM (25 mg/mL column).

After loading, a washing 1 step of performing treatment with a washing 1 buffer (buffer having a pH of 5.0 and including 20 mM acetate and 40 mM sodium chloride, 6.0 mS/cm) in an amount of 5 column volumes to attach the antibody which was not attached to the column and washing the remaining supernatant was performed.

Then, a washing 2 step was performed by setting a first buffer (pH 6.0, 30 mM acetate, 2.4 mS/cm) and a second buffer (buffer having a pH of 6.0 and including 30 mM acetate and 100 mM sodium chloride, 12.5 mS/cm) to a total amount of 15 column volumes to perform treatment with the first buffer and the second buffer at a ratio of 43 wt% and 57 wt%.

Here, a washing 2 step was performed in the form of a buffer in which the first buffer and the second buffer was mixed at a ratio of 43% and 57%, respectively.

Then, in order to perform a desorption step, the desorption step was performed while decreasing and increasing the first buffer and the second buffer in three steps of 40 wt%, 30 wt% and 20 wt%; and 60 wt%, 70 wt% and 80 wt%, respectively.

After each mixing was performed preferentially as in the above washing 2 step, such as two stages of gastric lavage, a three-step elution (desorption) step was performed using the mixed buffer.

Unlike a general method of the existing cation exchange chromatography, in the present invention, the washing step was significantly reduced to two steps compared to the technique in the related art, while simplifying the types of buffer solutions to three.

Furthermore, the step for performing desorption also enabled automation and simplification of the entire step by performing the reaction while adjusting only the ratio of the solution of the first buffer and the second buffer.

Further, in the cation exchange chromatography according to the present example, a flow rate which was 1.5-fold faster than the 180 cm/hr of the conventionally known methods was used. In the case of such a high flow rate, it is possible to have a higher production rate over time than that of the existing method.

Hereinafter, Table 2 specifically describes the process of purifying the isomeric antibody using the cation exchange resin Fractogel COO⁻ according to the present invention.

**[Table 2]**

| Procedure | Buffer step | Example 2-1 | Example 2-2 |
|---|---|---|---|
| Equilibration | pH 5.0, 20 mM acetate and 40 mM sodium chloride | 14 column volumes | 14 column volumes |
| Loading | Conductivity Con 6.4 mS/cm or less, adsorption capacity: 25 mg/column mL or less | | |
| Washing 1 | pH 5.0, 20 mM acetate and 40 mM sodium chloride | 5 column volumes | 5 column volumes |
| Washing 2 | pH 6.0, 30 mM acetate (43%); pH 6.0, 30 mM acetate and 100 mM sodium chloride (57%) | 15 column volumes | 15 column volumes |
| Desorption 1 | pH 6.0, 30 mM acetate (40%); pH 6.0, 30 mM acetate and 100 mM sodium chloride (60%) | 15 column volumes | 8 column volumes |
| Desorption 2 | pH 6.0, 30 mM acetate (30 %); pH 6.0, 30 mM acetate and 100 mM sodium chloride (70 %) | 9 column volumes | 15 column volumes |
| Desorption 3 | pH 6.0, 30 mM acetate (20 %); pH 6.0, 30 | 14 column | 9 column |
| | mM acetate and 100 mM sodium chloride (80 %) | volumes | volumes |
| Strip | 2 M NaCl | 2 column volumes | 2 column volumes |
| Column regeneration | 1 M NaOH | 3 column volumes | 3 column volumes |

Meanwhile, for comparison, elution was performed by adding a NaCl-free equilibration step in a manner similar to the prior Korean Patent No. 10-1498771. Specifically, treatment was performed with pH 5.0, 20 mM acetate and 40 mM sodium chloride in a total amount of 5 column volumes in washing 1 step; with pH 6.0, 30 mM acetate in a total amount of 10 column volumes in washing 2 step; with pH 6.0, 30 mM acetate and 50 mM sodium chloride in a total amount of 10 column volumes in desorption 1 step; with pH 6.0, 30 mM acetate in an amount of 1.5 column volumes in desorption 2 step; with pH 6.0, 30 mM acetate and 80 mM sodium chloride in an amount of 8 column volumes in desorption 3 step (Comparative Example).

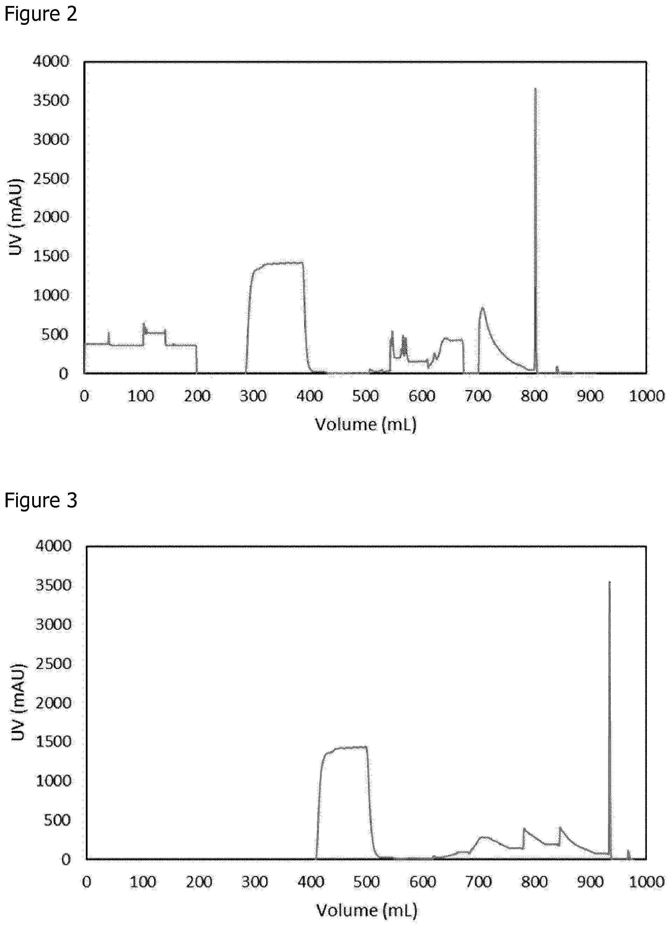

The experimental results are shown in Figures 2 to 4.

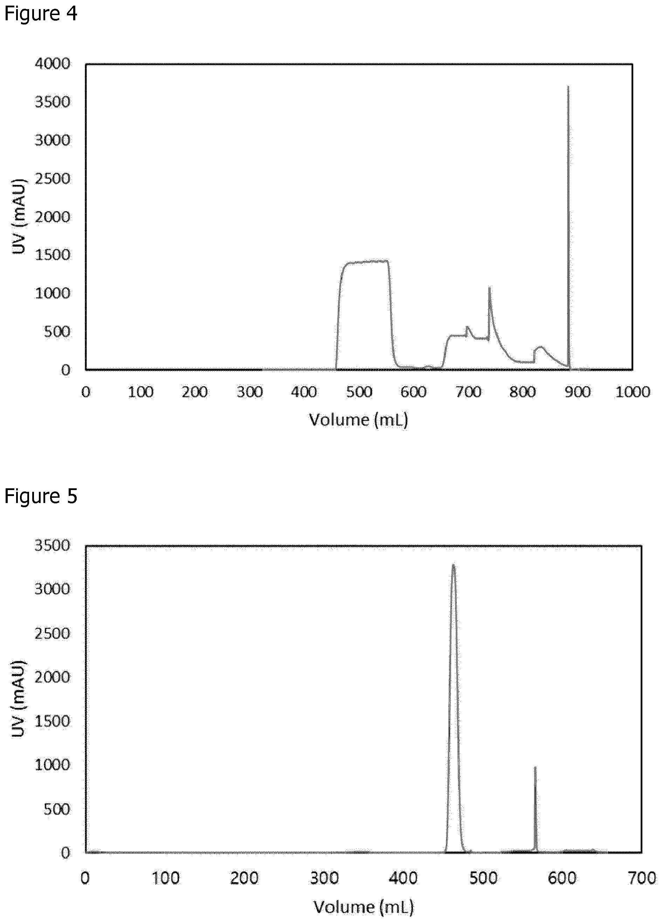

FIG. 2 illustrates the results of cation exchange chromatography according to the Comparative Example, FIG. 3 illustrates the results of Example 2-1 according to the present invention, and FIG. 4 illustrates the results of Example 2-2 according to the present invention.

The ratios and yields of the acidic isomeric antibody, the main active antibody and the basic isomeric antibody confirmed through the cation exchange chromatography are shown in the following Table 3.

**[Table 3]**

| | Example 2-1 | Example 2-2 | Comparative Example |
|---|---|---|---|
| Acidic isomeric antibody peak (%) | 16 to 19 | 14 to 19 | 15 to 16 |
| Main active antibody peak (%) | 66 to 69 | 65 to 69 | 68 |
| Basic isomeric antibody peak (%) | 13 to 15 | 13 to 19 | 16 |
| Yield (%) | 60 to 67 | 67 to 89 | 52 to 56 |

The purification process using the conditions of the cation exchange column as described above significantly increased the content of the main active antibody and also significantly increased the yield. In addition, process automation was made possible by using only three types of buffer solutions in collecting the protein to be eluted. Such advantages show an additional advantage of reducing the costs of buffer preparation and footprinting during antibody production.

The biggest advantage is that the convenience of obtaining antibodies in high yield is enhanced under established conditions, which can be suitably applied to process automation because the above three types of buffers are sequentially injected and proteins are collected based on column volume during elution.

### Example 3: Virus inactivation

Viruses were inactivated at a pH of 3.8 for 1 hour by adding a 1 M citric acid buffer to the primary eluates obtained in Examples 2-1 and 2-2. After the inactivation was completed, the pH of the sample was adjusted to 6.0 by adding a 2 M Trizma base buffer. The virus-inactivated sample was allowed to pass through a 0.2-µm filter and filtered.

### Example 4: Hydrophobic interaction resin

A process of increasing the purity of the antibody during antibody purification was performed using Phenyl Sepharose Fast Flow, which is a type of hydrophobic interaction chromatography (HIC).

Specifically, each eluate prepared by the method of Example 3 was used in order to perform HIC.

A method of performing elution by adsorbing the acetate at a concentration of 0.6 M citrate based on pH 6.0, and giving the elution buffer a concentration gradient of up to 5 column volumes during elution was used.

The base buffer in the cation exchange resin uses acetate with a pH of 6.0 or less because the use of a pH of 6.0 or less has an advantage in that the preparation process can be simplified in terms of buffer preparation.

In the elution method, the 5-column volume concentration gradient method showed excellent results in terms of yield, and in the case of a buffer, an acetate buffer with a pH of 6.0 was also excellent in terms of yield and antibody pH stability.

Specific conditions for the above hydrophobic interaction chromatography (HIC) are shown in the following Table 4.

**[Table 4]**

| |
|---|
| HIC loading solution: Fractogel COO⁻ (M) eluate+ |
| Eluate and equal volume of buffer including 60 mM acetate with pH 6.0 and 1.2 M citrate |
| Equilibration buffer: 30 mM acetate with pH 6.0 + 0.6 M citrate |
| Elution condition: 30 mM acetate |
| (5 column volume gradient elution) |

Hydrophobic interaction chromatography was performed under the above conditions, and the results are shown in FIG. 5.

### Example 5: Primary ultrafiltration

A Pellicon 3 Cassette (membrane) was equilibrated with a 25 mM Tris-HCI buffer (pH 7.5, 2.0 mS/cm) under conditions of Feed pressure ≤ 1 bar, and a process solution was concentrated to 10 mg/mL. Thereafter, the primary ultrafiltration was performed by performing buffer exchange such that the pH and conductivity of the process solution were 7.5 and ≤ 3.0 mS/cm.

### Example 6: Anion exchange resin chromatography

In the preparation method of the present invention, a process of preparing a population of antibodies having a higher purity was investigated using anion exchange resin chromatography.

Specifically, since the anion exchange column adsorbs cation-bearing proteins at the isoelectric point or higher, in the case of an antibody with an isoelectric point of 7 or higher (for example, in the case of adalimumab, the isoelectric point is 7 to 10, and in the case of Humira, the isoelectric point is approximately 8.4), when a neutral pH buffer is used, this antibody escapes to the flow-through without being attached to the anion exchange resin. Thus, the following experiments were conducted in order to investigate the conditions of the anion exchange resin and the buffer solution suitable for the preparation process of the present invention.

Specifically, in the present example, purification was performed using quaternary amine series Q Fast Flow (QFF, GE), which is frequently used as an anion exchange resin on a production scale. First, as a sample preparation for loading into the anion exchange resin, suitable conductivity and pH were prepared by substituting the buffer in a culture supernatant through a cation exchange column, a hydrophobic interaction column (HIC) and primary ultrafiltration. The purity, host cell protein content, residual DNA content and yield were confirmed under the conditions of 25 mM Tris HCI with a pH of 7.5 as a buffer.

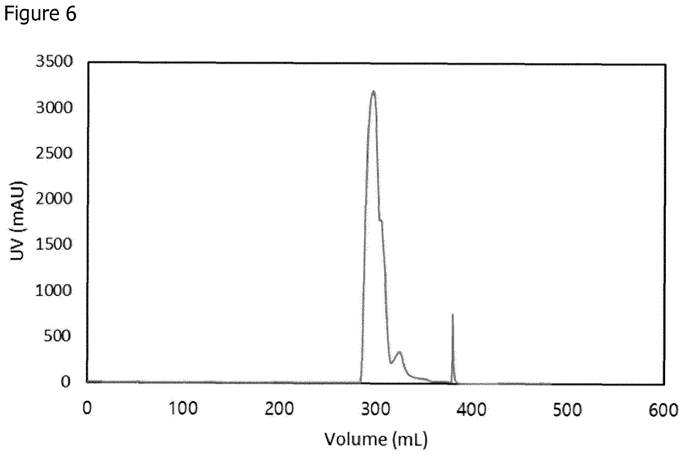

The results of chromatography using an anion exchange resin are shown in FIG. 6.

In the prepared population of antibodies, the purity was 100%, the HCP content was 6.8 ng/mg, the residual DNA content was 0.04 pg/mg, and the yield was 94 to 97%. As a result, it was suggested that the buffer in the anion exchange resin chromatography was a buffer including Tris HCI, and that the pH of 7 to 8 was advantageous for the preparation of the population of antibodies of the present invention.

### Example 7: Virus filtering, secondary ultrafiltration and diafiltration

The sample subjected to the anion exchange resin chromatography of Example 6 was filtered using a virus filter Modus 1.3 (Merck).

Then, secondary ultrafiltration was performed using a Pellicon 3 Cassette (membrane) filter. Specifically, a Pellicon 3 Cassette (membrane) was equilibrated with a 14 mM phosphate buffer (pH 5.2, 12.0 mS/cm) under conditions of Feed pressure ≤ 1 bar, and a process solution was concentrated to 55.5 mg/mL. Thereafter, buffer exchange was performed such that the pH and conductivity of the process solution were 5.2 and ≤ 11.0 mS/cm.

The virus filtering as described above performs virus filtering before the secondary ultrafiltration is performed, unlike the method in the existing prior patent Korean Patent No. 10-1498771. In the aforementioned prior patent, it is difficult to prepare a high-concentration drug because virus filtering is performed after performing primary ultrafiltration and secondary ultrafiltration. In contrast, the method according to the present invention has an advantage in that by first performing virus filtering before secondary ultrafiltration to reduce the loading amount in the secondary ultrafiltration step, protein quality is secured and simultaneously the yield loss is lowered, and a high-concentration drug is easily prepared.

### Example 8: Confirmation of changes in host-derived protein (HCP) and DNA content according to entire process in large batch

According to the procedures of Examples 1 to 7, the host-derived protein and DNA contents in the entire process were confirmed, and the results thereof are shown in the following Table 5.

In the case of the samples shown in the following Table 5, the results are for the samples obtained by the preparation process according to Example 2-2.

**[Table 5]**

| Adalimumab | Test Item | Volume (kg) | Concentration (mg/ml) | HCP content (ng/mg) | DNA content (pg/mg) |
|---|---|---|---|---|---|
| Day 13 of main culture | HCP, endotoxin, microorganisms | | 4.945268 | 17.3 | N/A |
| After main culture depth filter | HCP, endotoxin, microorganisms | 187.2 | 3.746587 | 20.5 | N/A |
| After main culture micro filtration | HCP, endotoxin, microorganisms | 217.9 | 3.788743 | 20.3 | N/A |
| CEX Pool (Example 2) | HCP, residual DNA | 624.7 | 0.7 | N/A | 0.1 |
| Virus Inactivation (Example 3) | HCP | 655.8 | 0.7 | N/A | 0.73 |
| HIC process eluate (Example 4) | HCP, Residual DNA | 172.6 | 2.4 | 29.4 | 0.09 |
| UF/DF1 process solution (Example 5) | HCP, Residual DNA | 48 | 8.1 | N/A | 0.11 |
| AEX eluate (Example 6) | HCP, residual DNA | 56.1 | 6.5 | 6.8 | 0.04 |
| VF process solution (Example 7 Virus filter) | HCP, residual DNA | 56.8 | 6.2 | 8.1 | 0.03 |
| UF/DF2 process solution (Example 7 Ultrafiltration) | HCP, residual DNA | 6.4 | 54.4 | 1.7 | 0.02 |
| DS (Example 7 Final population of antibodies) | | 7 | 49.2 | 1.6 | 0 |

As can be seen in Table 5, the HCP content and DNA content were confirmed for each step. As a result, the content of host cell-derived DNA was significantly reduced through the cation exchange chromatography described in Example 2-2. Then, virus inactivation was preferentially performed, and protein quality was secured and yield loss was lowered at the same time through ultrafiltration/diafiltration in a step immediately before formulation, facilitating high-concentration drug preparation. Furthermore, it was possible to prepare a population of antibodies in which the contents of host-derived protein and DNA were minimized through the above series of steps.

### Example 9: Confirmation of virus removal ability according to entire process in large batch

Among the entire process performed according to the procedures of Examples 1 to 7, the virus removal rate in the low pH treatment, anion exchange chromatography, and virus reduction filtration processes was confirmed, and the results thereof are shown in the following Table 6.

**[Table 6]**

| LOG REDUCTION FACTORS | | |
|---|---|---|
| LOW pH TREATMENT | RUN 1 | RUN 2 |
| Murine leukemia virus (MLV) | 6.57 ± 0.35 log₁₀ | 6.61 ± 0.40 log₁₀ |
| Pseudorabies virus (PRV) | ≥ 6.51 ± 0.34 log₁₀ | >6.51 ± 0.29 log₁₀ |

| ANION EXCHANGE CHROMATOGRAPHY | RUN 1 | RUN 2 |
|---|---|---|
| MLV | ≥ 6.47 ± 0.38 log₁₀ | ≥ 6.55 ± 0.28 log₁₀ |
| PRV | ≥ 6.28 ± 0.34 log₁₀ | ≥ 6.46 ± 0.38 log₁₀ |
| Reovirus type-3 (Reo 3) | ≥ 8.47 ± 0.29 log₁₀ | ≥ 8.21 ± 0.31 log₁₀ |
| Mouse minute virus (MMV) | ≥ 6.49 ± 0.25 log₁₀ | ≥ 6.49 ± 0.32 log₁₀ |

| VIRUS REDUCTION FILTRATION | RUN 1 | RUN 2 |
|---|---|---|
| MLV | ≥ 5.52 ± 0.36 log₁₀ | ≥ 5.44 ± 0.25 log₁₀ |
| PRV | ≥ 5.60 ± 0.25 log₁₀ | ≥ 5.52 ± 0.31 log₁₀ |
| Reo 3 | ≥ 5.60 ± 0.31 log₁₀ | ≥ 5.34 ± 0.30 log₁₀ |
| MMV | ≥ 6.05 ± 0.23 log₁₀ | ≥ 6.40 ± 0.36 log₁₀ |

As can be seen in Table 6, the inactivation and removal ability of MLV, PRV, Reo3, and MMV viruses were confirmed for each step. The results show that there is almost no expression of virus in the population of antibodies according to the process according to the present invention. In particular, in consideration of the virus inactivation and removal ability in each of the steps, it is shown that even though there is unexpected virus expression or contamination in the purification process according to the present invention, it can be removed.

From the foregoing description, it will be understood by those skilled in the art to which the present invention pertains that the present invention can be implemented in other concrete forms without modifying the technical spirit or essential features of the present invention. In this regard, it should be understood that the above-described embodiments are only exemplary in all aspects and are not restrictive. The scope of the present invention is represented by the claims to be described below rather than the detailed description, and it should be interpreted that the meaning and scope of the claims and all the changes or modified forms derived from the equivalent concepts thereto fall within the scope of the present invention.

## Claims

1. A method of preparing a population of antibodies, the method comprising:
(a) a step of removing a host cell protein (HCP) and an isomeric antibody from a sample comprising a mixed solution of antibodies, comprising loading the sample comprising the mixed solution of antibodies into an equilibrated cation exchange column, washing the cation exchange column, and then eluting antibodies bound to the column with an elution buffer;
(b) a step of removing the host cell protein (HCP) and the residual DNA from an antibody eluate, comprising loading a sample obtained by mixing a salt with an antibody eluate eluted in Step (a) into a hydrophobic interaction column and eluting antibodies bound to the column with an elution buffer;
(c) a step of removing the host cell protein (HCP) and the residual DNA, including collecting a flow-through by allowing an antibody eluate from which the host cell protein (HCP) and the residual DNA in Step (b) have been removed to pass through an anion exchange column;
(d) a step of removing viruses by allowing the flow-through in Step (c) to pass through a virus filter; and
(e) a step of concentrating the antibody eluate eluted in Step (d), performing buffer exchange and preparing a population of antibodies containing the residual DNA and the host cell protein at a concentration of 10 ppb and 10 ppm or less, respectively.

2. The method of claim 1, wherein a sample comprising a mixed solution of antibodies in Step (a) is prepared by a method comprising a step of removing a precipitated precipitate by adjusting the pH of a culture supernatant to 4 to 6.

3. The method of claim 1, wherein the sample comprising the mixed solution of antibodies in Step (a) has a conductivity of 5 mS/cm to 7 mS/cm.

4. The method of claim 1, wherein the antibody has an isoelectric point of 7 to 10.

5. The method of claim 1, wherein the antibody is adalimumab.

6. The method of claim 1, wherein the antibody eluate eluted in Step (a) comprises 60% or more of a main active antibody, 20% or less of an acidic isomeric antibody, and 20% or less of a basic isomeric antibody.

7. The method of claim 1, wherein the step of loading the sample comprising the mixed solution of antibodies into an equilibrated cation exchange column comprises a step of loading the sample comprising the mixed solution of antibodies into a Fractogel COO⁻ column equilibrated with an equilibration buffer having a pH of 4.5 to 5.5 and comprising 15 mM to 30 mM acetate and 35 mM to 45 mM sodium chloride.

8. The method of claim 1, wherein the step of washing the cation exchange column comprises:
1) a first washing step of washing the column with a buffer having a pH of 4.5 to 5.5 and comprising 15 mM to 30 mM acetate and 35 mM to 45 mM sodium chloride;
2) a second washing step of washing the column with a buffer having a pH of 5.5 to 6.5 and comprising 25 mM to 35 mM acetate and 55 to 59 mM sodium chloride.

9. The method of claim 8, wherein the buffer in the second washing step is prepared so as to have a sodium chloride molar concentration of 55 to 59 mM by mixing a buffer having a pH of 5.5 to 6.5 and comprising 25 mM to 35 mM acetate and a buffer having a pH of 5.5 to 6.5 and comprising 25 mM to 35 mM acetate and sodium chloride having a predetermined molar concentration.

10. The method of claim 1, wherein the step of eluting the antibody bound to the column with the elution buffer comprises:
1) a first elution step of eluting an antibody with a buffer having a pH of 5.5 to 6.5 and comprising 25 mM to 35 mM acetate and 57 to 63 mM sodium chloride;
2) a second elution step of eluting the antibody with a buffer having a pH of 5.5 to 6.5 and comprising 25 mM to 35 mM acetate and 67 to 73 mM sodium chloride; and
3) a third elution step of eluting the antibody with a buffer having a pH of 5.5 to 6.5 and comprising 25 mM to 35 mM acetate and 77 to 83 mM sodium chloride.

11. The method of claim 10, wherein the buffers in the first, second and third elution steps are prepared so as to have predetermined sodium chloride molar concentrations in the first, second and third elution steps by mixing a buffer having a pH of 5.5 to 6.5 and comprising 25 mM to 35 mM acetate and a buffer having a pH of 5.5 to 6.5 and comprising 25 mM to 35 mM acetate and sodium chloride having a predetermined molar concentration.

12. The method of claim 1, wherein Step (a) comprises:
1) a step of loading a mixed solution of antibodies into a Fractogel COO⁻ cation exchange column equilibrated with an equilibration buffer having a pH of 4.5 to 5.5 and comprising 15 mM to 30 mM acetate and 35 mM to 45 mM sodium chloride;
2) a first washing step of washing the column with a buffer having a pH of 4.5 to 5.5 and comprising 15 mM to 30 mM acetate and 35 mM to 45 mM sodium chloride;
3) a second washing step of washing the column with a buffer having a pH of 5.5 to 6.5 and comprising 25 mM to 35 mM acetate and 55 mM to 59 mM sodium chloride;
4) a first elution step of eluting an antibody with a buffer having a pH of 5.5 to 6.5 and comprising 25 mM to 35 mM acetate and 57 to 63 mM sodium chloride;
5) a second elution step of eluting the antibody with a buffer having a pH of 5.5 to 6.5 and comprising 25 mM to 35 mM acetate and 67 to 73 mM sodium chloride; and
6) a third elution step of eluting the antibody with a buffer having a pH of 5.5 to 6.5 and comprising 25 mM to 35 mM acetate and 77 to 83 mM sodium chloride.

13. The method of claim 1, wherein Step (b) elutes the antibody by a concentration gradient method.

14. The method of claim 13, wherein the concentration gradient method comprises a step of eluting an antibody by loading a sample in which the antibody eluate eluted in Step (a) is adjusted to a citrate concentration that is the same as that of an equilibration buffer into a hydrophobic interaction column equilibrated with an equilibration buffer comprising 25 mM to 35 mM acetate (pH of 5.5 to 6.5) and 0.3 M to 1.0 M citrate and applying an elution buffer having a pH of 5.5 to 6.5 and comprising 25 mM to 35 mM acetate in a concentration gradient manner.

15. The method of claim 1, wherein the hydrophobic interaction column in Step (b) is a phenyl sepharose column.

16. The method of claim 1, wherein the anion exchange column in Step (c) is equilibrated with an equilibration buffer having a pH of 7.0 to 8.0 before injection of the sample.

17. The method of claim 16, wherein the equilibration buffer comprises Tris-HCI having a pH of 7.0 to 8.0.

18. The method of claim 1, wherein the anion exchange column in Step (c) is a Q Fast Flow column.

19. A population of antibodies prepared by the method of claim 1, wherein the population of antibodies comprises 60% or more of a main active antibody.

20. A population of antibodies prepared by the method of claim 1, wherein a concentration of the residual DNA and the host cell protein is 0.1 ppb and 5 ppm or less, respectively.
